# EUROPÄISCHE PATENTSCHRIFT

(11) **EP 1 062 970 B1**
(45) Veröffentlichungstag und Bekanntmachung des Hinweises auf die Patenterteilung: **09.11.2005**
(21) Anmeldenummer: 00250202.9
(22) Anmeldetag: 23.06.2000
(51) Int. Cl.: A61N 1/05, A61B 5/0428, A61N 1/36

(54) **Elektrodenanordnung**
Electrode assembly
Ensemble électrode

(30) Priorität: 25.06.1999 DE 19930271
(43) Veröffentlichungstag der Anmeldung: 27.12.2000
(73) Patentinhaber: BIOTRONIK Mess- und Therapiegeräte GmbH & Co Ingenieurbüro Berlin, 12359 Berlin (DE)
(72) Erfinder: Witte, Joachim, 10409 Berlin (DE)
(74) Vertreter: Eisenführ, Speiser & Partner

(56) Entgegenhaltungen:
- FR-A- 2 566 201
- US-A- 4 628 934
- US-A- 5 018 523
- US-A- 5 469 000

## Beschreibung

Die Erfindung betrifft eine implantierbare Elektrodenanordnung, die eine Elektrodenleitung mit mehreren elektrisch leitenden Oberflächenbereichen im Bereich des distalen Endes zur Abgabe elektrischer Signale an ein Herz und/oder zur Aufnahme von Signalen von einem Herzen umfaßt, die über die Elektrodenleitung elektrisch mit einer elektrische Signale aufnehmenden und/oder Impulse abgebenden kardioelektrischen Vorrichtung für einen Defibrillator oder Herzschrittmacher verbindbar sind.

Elektrodenanordnungen mit einer Elektrodenleitung und mehreren elektrisch leitenden Oberflächenbereichen beispielsweise von Tip- oder Ringelelektroden am distalen Ende der Elektrodenleitung sind beispielsweise aus der EP 0 571 797 bekannt. Eine Elektrodenanordnung gemäß dem Oberbegriff von Anspruch 1 ist aus US 4,628,939 bekannt. Bei den bekannten Elektrodenanordnungen sind die als Stimulations- oder Abfühlelektroden dienenden elektrisch leitenden Oberflächenabschnitte einzeln mittels elektrischer Leitungen, die in der Elektrodenleitung verlaufen, mit einem Herzschrittmacher oder Defibrillator verbunden. In den genannten Druckschriften ist auch jeweils beschrieben, daß von der Vielzahl der Elektroden oder Elektrodenkombinationen die jeweils bestgeeigneten ausgewählt werden, um diese beispielsweise zur Stimulation eines menschlichen Herzens zu verwenden. Nachteilig an den bekannten Elektrodenanordnungen ist, daß sie regelmäßig nur zusammen mit speziell angepaßten Herzschrittmachern oder Defibrillatoren verwendet werden können, die es erlauben, am proximalen Ende der Elektrodenleitung sämtliche Zuleitungen zu kontaktieren, die zu den elektrisch leitenden Oberflächenbereichen führen.

Üblich sind Elektrodenanordnungen, bei denen in der Elektrodenleitung nur ein oder zwei elektrische Leiter verlaufen, je nachdem, ob die Elektrodenanordnung zur unipolaren oder zur bipolaren Stimulation vorgesehen ist.

Elektrodenleitungen mit einer Eindrahtverbindung zwischen dem proximalen Ende der Elektrodenleitung und den elektrisch leitenden Oberflächenbereichen am distalen Ende der Elektrodenleitung sind für die unipolare Stimulation geeignet, bei der Stimulationsimpulse zwischen den elektrisch leitenden Oberflächenbereichen am distalen Ende der Elektrodenleitung und eine Neutralelektrode wie beispielsweise einem Gehäuse des Herzschrittmachers abgegeben werden. Daneben ist auch die bipolare Stimulation bekannt, bei der die Stimulationsenergie zwischen verschiedenen der elektrisch leitenden Oberflächenbereiche am distalen Ende der Elektrodenleitung abgegeben wird. Für die bipolare Stimulation ist in der Elektrodenleitung eine Zweidrahtverbindung zwischen proximalem und distalem Ende vorgesehen, die über zwei separate elektrische Leitungen hergestellt wird.

Ziel der Erfindung ist es, die Vorteile von Elektrodenanordnungen mit einer Mehrzahl von elektrisch leitenden und einzeln ansteuerbaren Oberflächenbereichen auch für Herzschrittmacher oder Defibrillatoren mit herkömmlichen Ein- oder Zweidrahtanschlüssen verfügbar zu machen.

Dieses Ziel wird erfindungsgemäß mit einer Elektrodenanordnung der eingangs genannten Art erreicht, die sich durch Schaltmittel auszeichnet, die derart angeordnet ausgestaltet sind, daß die Verbindung zwischen einzelnen der elektrisch leitenden Oberflächenbereiche und der kardioelektrischen Vorrichtungen im Bereich der Elektrodenleitung dauerhaft ein- oder ausschaltbar ist. Eine solche Elektrodenanordnung erlaubt es, zunächst beliebige der elektrisch leitenden Oberflächenbereiche durch die Aufnahme oder Abgabe elektrischer Signale auszuwählen und anschließend dauerhaft über eine herkömmliche Eindraht- oder Zweidrahtleitung in der Elektrodenleitung mit der kardioelektrischen Vorrichtung wie einem Defibrillator oder Herzschrittmacher zu verwenden.

Zu diesem Zweck ist es vorteilhaft, wenn die Schaltmittel so ausgestaltet sind, daß sie ihren Schaltzustand im stromlosen Zustand beibehalten.

In einer bevorzugten Ausführungsvariante sind die Schaltmittel über die Elektrodenleitung elektrisch ansteuerbar. Dazu weistdie Elektrodenleitung vorzugsweise einen Decoder auf, der einerseits mit den Schaltmitteln und andererseits an mindestens eine elektrische Leitung - beispielsweise die übliche Eindraht- oder Zweidrahtleitung, die die elektrisch leitenden Oberflächenbereiche mit dem Herzschrittmacher verbindet - verbunden ist. Der Decoder ist dazu so gestaltet, daß er über diese elektrische Leitung Steuersignale empfangen und die Schaltmittel in Abhängigkeit der Steuersignale einzeln ansteuern kann. Eine derartige Elektrodenanordnung braucht im Minimalfall nur einen einzigen elektrischen Leiter, der vom proximalen Ende der Elektrodenleitung zu den Schaltmitteln, dem Decoder und den elektrisch leitenden Oberflächenbereichen am distalen Ende der Elektrodenleitung führt. Das Bezugspotential für die Steuersignale kann dann beispielsweise über eine Neutralelektrode mit dem Gehäuse des Schrittmachers hergestellt werden. Nach dem Ermitteln der bestgeeigneten Elektroden oder Elektrodenkombinationen können diese dann mit Hilfe des Decoders und der Schaltmittel dauerhaft über die eine elektrische Leitung in der Elektrodenleitung mit beispielsweise einem Herzschrittmacher verbunden werden. Dieses kann ein üblicher Herzschrittmacher sein, für den sich dann ebenfalls die Vorteile der individuell ermittelten, bestgeeigneten Elektrodenkonfigurationen ergeben.

Für das dauerhafte Verbinden der elektrisch leitenden Oberflächenabschnitte mit dem elektrischen Leiter in der Elektrodenleitung oder umgekehrt für das dauerhafte Trennen einzelner Oberflächenbereiche von dem elektrischen Leiter können elektrische Bauelemente eingesetzt werden, die zum Schalten oder Trennen der Verbindung eine größere elektrische Leistung benötigen, als beispielsweise über den Eindrahtleiter und die Neutralelektrode als Gegenelektrode zur Verfügung gestellt werden kann. Um solche elektrischen Bauelemente einsetzen zu können, umfaßt die Elektrodenanordnung vorzugweise einen Energiespeicher, der mit den Schaltmitteln und/oder dem Decoder verbunden und mit einer geringeren Leistung über die Elektrodenleitung aufladbar ist, als er über die Schaltmittel und/oder den Decoder abgeben kann. Vorzugsweise ist der Energiespeicher ein elektrischer Kondensator, der beispielsweise via Feindrahtleitung und Neutralelektrode hochohmig geladen wird, und die auf diese Weise angesammelte elektrische Energie kurzfristig zum Schalten der Schaltelemente abgeben kann. Dieses kann beispielsweise das Durchschalten eines Leistungsfeldtransistors oder das Durchbrennen eines Thyristors sein. Auf diese Weise wird eine Schaltverbindung dauerhaft hergestellt bzw. dauerhaft getrennt. Der letztgenannte Fall ist der bevorzugte, bei dem die Schaltelemente so gestaltet sind, daß zum Ausschalten einer Verbindung zwischen der Elektrodenleitung und einem elektrisch leitenden Oberflächenbereich die Verbindung dauerhaft zerstörbar ist. Der Energiespeicher ist vorzugsweise zur Gabe der dazu benötigten Energie gestaltet.

Auch wenn der elektrische Energiespeicher insbesondere bei solchen Elektroden mit einer Eindrahtverbindung zwischen proximalem und distalem Ende wegen der zum Laden des Kondensators gering zu haltenden Leistung besondere Vorteile aufweist, damit betroffenes Herzgewebe nicht bereits beim Laden des Energiespeichers stimuliert wird, kann ein solcher Energiespeicher ebenso vorteilhaft bei einer Elektrodenleitung mit einer Zweidrahtverbindung zwischen proximalem und distalem Ende verwendet werden, mit der prinzipbedingt größere Leistungen zum Energiespeicher übertragen werden können, als bei einer Elektrodenleitung mit nur einer Eindrahtverbindung.

Insbesondere für das Austesten einzelner Oberflächenbereiche oder Kombinationen wird eine Elektrodenanordnung bevorzugt, die Steuermittel enthält, die eingangsseitig über zumindest eine elektrische Leitung für Signale zu oder von den elektrisch leitenden Oberflächenbereichen an die kardioelektrische Vorrichtung anschließbar und ausgangsseitig mit den elektrisch leitenden Oberflächenbereichen verbunden sind, die derart gestaltet sind, daß sie durch über die elektrische Leitung eingehende Steuersignale derart steuerbar sind, daß einzelne Oberflächenbereiche oder Kombinationen von Oberflächenbereichen mit der kardioelektrischen Vorrichtung zum Abgeben und Aufnehmen von Signalen für das Ermitteln für die dauerhafte Verbindung mit den für die kardioelektrische Vorrichtung geeigneten elektrisch leitenden Oberflächenabschnitten verbindbar sind. Die letztgenannte Variante betrifft also nicht das dauerhafte Verbinden der elektrisch leitenden Oberflächenbereiche mitderkardioelektrischen Vorrichtung, sondern das testweise Verbinden der Oberflächenbereiche mit der Vorrichtung vor dem dauerhaften Einstellen einer geeigneten Elektrodenkombination.

Eine alternativ bevorzugte Elektrodenanordnung zeichnet sich durch einen relativ zur Elektrodenleitung längs- oder drehbeweglich geführten Mandrin aus, der Betätigungselemente für mechanische Schaltelemente aufweist, mit denen die Verbindung zwischen einzelnen der elektrisch leitenden Oberflächenbereiche und der kardioelektrischen Vorrichtung im Bereich der Elektrodenleitung dauerhaft ein- oder ausschaltbar ist. Mit Hilfe eines derartigen Mandrins und der Schaltelemente können die Schaltelemente in der Elektrodenleitung so verschoben oder gedreht werden, daß sie wahlweise einen Kontakt zwischen einzelnen oder mehreren der elektrisch leitenden Oberflächenbereiche und einer elektrischen Leitung in der Elektrodenleitung herstellen oder unterbrechen. Da sich die entsprechenden mechanischen Schaltelemente nicht ohne Betätigung durch den Mandrin umstellen, wird auf diese Weise eine Verbindung zwischen den elektrisch leitenden Oberflächenbereichen und dem elektrischen Leiter in der Elektrodenleitung dauerhaft hergestellt oder unterbrochen.

Im Zusammenhang mit der letztgenannten Ausführungsvariante wird eine Elektrodenanordnung bevorzugt, bei der die Schaltelemente im Bereich des distalen Endes der Elektrodenleitung längs- oder drehbeweglich angeordnet sind und mit zumindest einer elektrischen Leitung in der Elektrodenleitung in elektrischem Kontakt stehen und weiterhin Kontaktbereiche aufweisen, die in einer von zumindest zwei möglichen Stellungen der Schaltelemente einen mit mindestens einem elektrischen leitenden Oberflächenbereich in Verbindung stehenden Gegenkontakt kontaktieren und weiterhin Schaltungen aufweisen, mit denen ein Schaltelement mittels entsprechender Gegennocken am Mandrin derart mittels des Mandrin bewegbar ist, daß die Schaltmittel mittels des Mandrin jeweils in eine einen Oberflächenbereich mitder kardioelelektrischen Vorrichtung verbindende und eine einen Oberflächenbereich von der kardioelektrischen Vorrichtung trennende Stellung bewegbar sind. Das Prinzip zweier Nocken, die miteinander in Eingriff zu bringen sind, um ein mechanisches Element zu drehen und längs zu verschieben, ist dem Fachmann in allen seinen Varianten bekannt und leicht auf einen Mandrin mit mehreren Betätigungsnocken zu übertragen, bei dem die Betätigungsnocken so zueinander versetzt sind, daß alle mechanischen Schaltelemente einzeln und unabhängig voneinander mit einem Mandrin verschoben bzw. gedreht werden können.

Die beschriebene Elektrodenanordnung kann mit einem verfahren welches nicht Teil der Erfindung ist betrieben werden, bei dem zunächst für die Signalaufnahme und/oder Stimulation geeignete Oberflächenbereiche ermittelt werden und anschließend die geeigneten Oberflächenbereiche dauerhaft mit zumindest einer elektrischen Leitung der Elektrodenleitung verbunden werden, während die übrigen Oberflächenbereiche dauerhaft von der elektrischen Leitung getrennt werden. Das Verfahren ist somit zweistufig, zunächst werden vorläufige Verbindungen zwischen den elektrisch leitenden Oberflächenbereichen und beispielsweise einem Herzschrittmacher hergestellt, um die elektrisch leitenden Oberflächenbereiche oder Kombinationen hinsichtlich ihrer Eignung zu testen. Dazu dienen die bereits genannten Steuermittel. Anschließend werden die bestgeeigneten Oberflächenbereiche dauerhaft mit einer zum proximalen Ende der Elektrodenleitung führenden elektrischen Leitung verbunden oder dementsprechend alle außer den bestgeeigneten Oberflächenbereichen dauerhaft von der elektrischen Leitung getrennt.

Die Erfindung soll nun anhand von Ausführungsbeispielen mit Hilfe der Figuren näher erläutert werden. Diese zeigen:
- Figur 1: Das distale Ende einer Elektrodenleitung mit elektrisch leitenden Oberflächenbereichen als Elektroden;
- Figur 2: Einen Längsschnitt durch einen Teil der in Figur 1 abgebildeten Elektrodenleitung;
- Figur 3a und 3b: Einen Längsschnitt durch einen Teil der in Figur 1 abgebildeten Elektrodenleitung in einer alternativen Ausführungsvariante und
- Figur 4: Einen Querschnitt durch die Ausführungsvariante in Figur 3 im Bereich einer Ringelektrode.

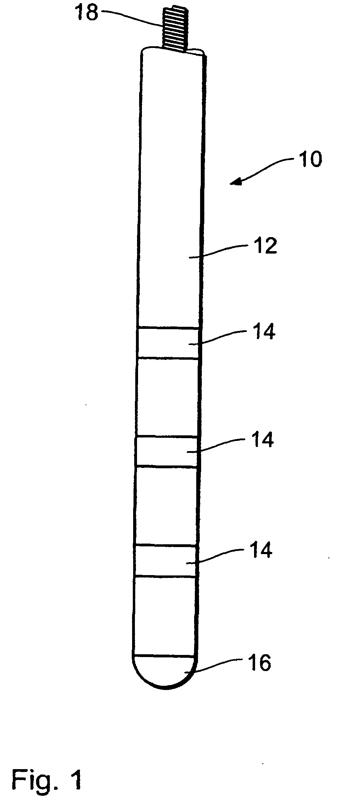

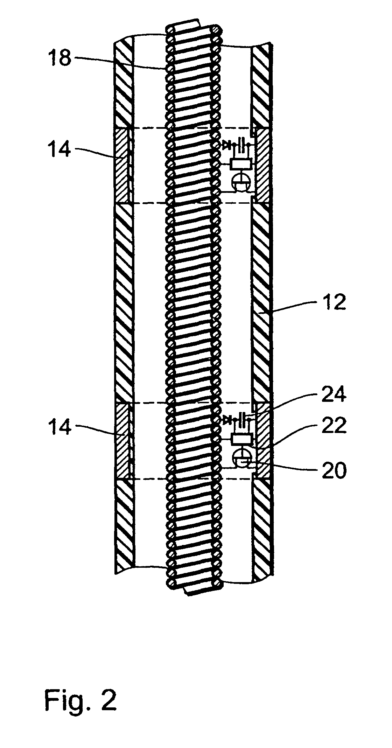

Das in Figur 1 dargestellte distale Ende umfaßt eine Elektrodenleitung 10 mit einer isolierenden, Hülle 12 und darin eingelassenen Ringelektroden 14 sowie einer Tipelektrode 16 am äußersten Ende der Elektrodenleitung 10. Im Inneren der Elektrodenleitung 10 verläuft eine gewendelte elektrische Leitung 18, die zum Übertragen elektrischer Signale von und zu den Elektroden 14 und 16 dient. Die Elektroden 14 und 16 können beispielsweise aus Metall bestehen, wesentlich ist, daß die Elektroden 14 und 16 elektrisch leitende Oberflächenbereiche der Elektrodenleitung 10 darstellen, die zumindest teilweise mit der elektrischen Leitung 18 verbindbar sind. Der Aufbau der Elektrodenleitung 10 ist dem in Figur 2 abgebildeten Längsschnitt durch die Elektrodenleitung 10 im Bereich zweier Ringelektroden 14 zu entnehmen. Zu erkennen ist die gewendelte elektrische Leitung 18, die innerhalb der isolierenden Hülle 12 verläuft. Zwischen der Leitung 18 und jeder Ringelektrode 14 sind folgende elektrische Bauelemente angeordnet: jeweils ein Feldeffekttransistor 20, ein Decoder bzw. eine Steuereinheit 22, sowie ein Kondensator 24. Die Feldeffekttransistoren 20 sind Leistungstransistoren mit geringerem Durchgangswiderstand im durchgeschalteten Zustand. Sie stellen jeweils ein Schaltmittel zum Verbinden oder Unterbrechen einer elektrischen Verbindung zwischen der Leitung 18 und einer jeweiligen Ringelektrode 14 dar. Angesteuert werden die Feldeffekttransistoren 20 von der Steuereinheit bzw. dem Decoder 22. Die Steuereinheit bzw. der Decoder 22 haben zwei Steuereingänge, von denen einer mit der elektrischen Leitung 18 und der andere mit jeweils einer Ringelektrode 14 verbunden ist. Über die Steuereingänge kann die Steuereinheit 22 Steuersignale aufnehmen, die beispielsweise von einem Herzschrittmacher über die elektrische Leitung 18 sowie einer Neutralelektrode wie das Herzschrittmachergehäuse abgegeben werden und sich dementsprechend als Potentialfolge darstellen, die zwischen der Leitung 18 und einer Ringelektrode 14 liegt. Die Ringelektrode 14 ist dabei über einem Körper, in den sie eingesetzt ist, mit der Neutralelektrode verbunden.

Der Energieversorgung der Steuereinheit 22 dient ein Kondensator 24, der ebenfalls zwischen der Leitung 18 und jeweils einer Ringelektrode 14 angeordnet ist. Dieser Kondensator 24 wird über die elektrische Leitung einerseits sowie andererseits über die Ringelektrode 14 und die mit dieser korrespondierenden Neutralelektrode (nicht dargestellt) vom Herzschrittmacher mit Energie versorgt und hochohmig geladen.

Über entsprechende Steuersignale können beliebige der Elektroden 14 und 16 mittels der Steuereinheiten 22 und der Feldeffekttransistoren 20 mit der Leitung 18 verbunden oder von ihr getrennt werden.

Anstelle der in Figur 2 dargestellten Steuer- und Schaltmittel können beispielsweise auch EPROMs, also elektrisch programmierbare Lesespeicher, verwendet werden, die über entsprechende Steuersignale konfiguriert werden und die gewünschten Verbindungen zu den Elektroden 14 und 16 und der Leitung 18 herstellen.

Alternativ ist es auch möglich, zwischen den Elektroden 14 und 16 und der Leitung 18 einfache Drahtverbindungen vorzusehen, die bei Bedarf von entsprechenden Steuermitteln mit Hilfe von in einem Kondensator gespeicherter elektrischer Energie durchgebrannt werden, um eine Verbindung zwischen einer Elektrode 14 oder 16 und der Leitung 18 dauerhaft zu unterbrechen.

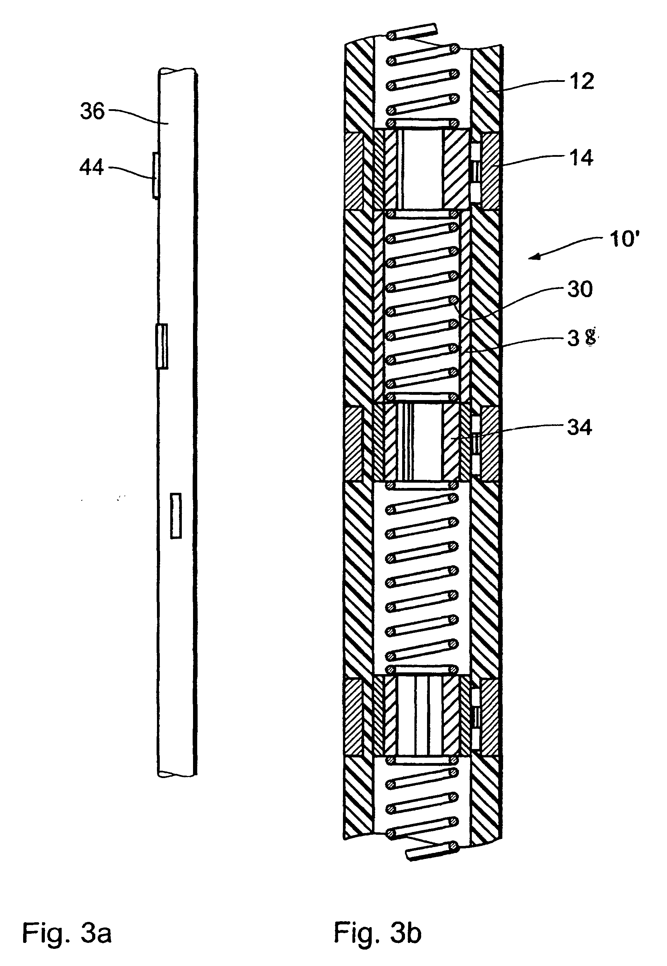

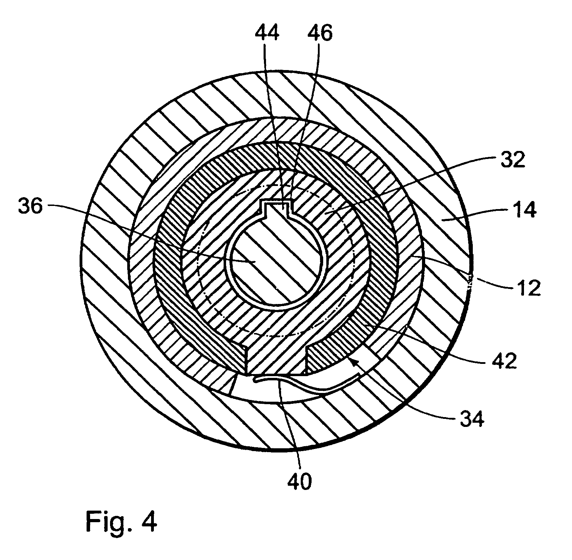

Alternativ zu den beschriebenen elektrischen Varianten zum dauerhaften Herstellen oder Unterbrechen einer elektrischen Verbindung zwischen den Elektroden 14 und 16 an der Leitung 18 ist auch die in den Figuren 3a und 3b sowie 4 dargestellte mechanische Variante vorgesehen. Wie der Schnitt durch die Elektrodenleitung 10 in Figur 3b zeigt, ist der äußere Aufbau der Elektrodenleitung 10 identisch mit den zuvor beschriebenen Varianten: in einer isolierenden Hülle 12 sind Ringelektroden 14 in Form von Metallringen eingelassen. Die elektrische Leitung ist im Bereich des abgebildeten distalen Endes der Elektrodenleitung nicht als durchgehende Drahtwendel ausgebildet, sondern wird abschnittsweise von Druckfedern 30 und jeweils einem Metallkörper 32 mechanischer Schaltelemente 34 gebildet.

Zum besseren Verständnis der Figuren 3a und 3b ist anzumerken, daß diese die Elektrodenleitung 10 der Übersichtlichkeit halber in teildemontiertem Zustand zeigen. Im montierten Zustand verläuft der in Figur 3a abgebildete Mandrin 36 koaxial zur Elektrodenleitung 10' in derselben und durchdringt somit die Schaltelemente 34 und verläuft innerhalb der Druckfedern 30.

Wie Figur 3b zu entnehmen ist, sind die Schaltelemente 34 durch Distanzhülsen 38 bezüglich ihrer axialen Position in der Elektrodenleitung 10' so festgelegt, daß die Schaltelemente 34 den Ringelelektroden 14 auf deren Innenseite gegenüberliegen. Die Schaltelemente 34 sind um die Längsachse der Elektrodenleitung 10' drehbar angeordnet. Jeweils ein Schaltkontakt 40 der Ringelektroden 14 kontaktiert die Außenfläche der Schaltelemente 34.

Wie insbesondere der Figur 4 zu entnehmen ist, die einen Querschnitt durch die Elektrodenleitung 10' im Bereich einer Ringelektrode 14 und eines Schaltelementes 34 darstellt, kann der Schleifkontakt 40 einen elektrischen Kontakt zwischen dem metallenen Körper 32 des Schaltelementes 34 und der Ringelektrode 14 herstellen, wenn sich das Schaltelement 34 in einer dazu geeigneten Winkelstellung befindet. In anderen als in der in Figur 4 dargestellten Winkelstellung des Schaltelementes 34 verhindert eine Isolierung 42 auf der Außenfläche des Schaltelementes 34 einen elektrischen Kontakt zwischen dem metallenen Körper 32 des Schaltelementes 34 und der Ringelektrode 14. Auf diese Weise besteht in der in Figur 4 dargestellten Stellung des Schaltelementes 34 über die Druckfedern 30 und dem metallenen Körper 32 der Schaltelemente 34 sowie den Schleifkontakt 40 eine elektrische Verbindung zwischen der elektrischen Leitung in der Elektrodenleitung 10' und der Ringelektrode 14. Befindet sich das Schaltelement 34 in einer anderen als der dargestellten Winkelstellung, ist die elektrische Verbindung zwischen der elektrischen Leitung und der Elektrode 14 unterbrochen.

Zum Auswählen der Winkel- und damit der Schaltstellungen der Schaltelemente 34 dient der Mandrin 36, der dazu in der Elektrodenleitung 10' drehbar und längsverschieblich gelagert ist. Der Mandrin 36 besitzt Schaltnocken 44, die jeweils in eine Nut 46 in dem metallenen Körper 32 des Schaltelementes 34 in Eingriff gebracht werden können. Dies geschieht durch Längsverschieben des Mandrins 36. Der Abstand der Schaltnocken 44 in Längsrichtung des Mandrins 36 ist so gewählt, daß jeweils nur eine Schaltnocke in eine Nut 46 eines Schaltelementes 34 eingreift. Dasjenige Schaltelement 34, in das Schaltnocke 44 des Mandrins 36 eingreift, kann durch Drehen des Mandrins 36 um dessen Längsachse in eine Winkelstellung gebracht werden und somit in eine Endverbindung zur jeweiligen Ringelektorde 14 herstellende oder trennende Winkelstellung.

## Patentansprüche

1. Implantierbare Elektrodenanordnung, umfassend eineElektrodenleitung (10, 10') mit einer, isolierenden Hülle (12) und mit mehreren elektrisch leitenden Oberflächenbereichen (14, 16) im Bereich des distalen Endes der Elektrodenleitung (10, 10') zur Abgabe elektrischer Signale an ein Herz und/oder zur Aufnahme von Signalen von einem Herzen, die über die Elektrodenleitung (10, 10') elektrisch mit einer elektrische Signale aufnehmenden und/oder Impulse abgebenden kardioelektrischen Vorrichtung wie einem Defibrillator oder einem Herzschrittmacher verbindbar sind, sowie mit Schaltmitteln (20, 22; 34), die derart gestaltet sind, daß die Verbindung zwischen einzelnen der elektrisch leitenden Oberflächenbereiche (14, 16)und der kardioelektrischen Vorrichtung im Bereich der Elektrodenleitung (10, 10') dauerhaft ein- oder ausschaltbar ist, **dadurch gekennzeichnet, dass** die Schaltmittel (20, 22; 34) innerhalb der Elektroden leitung (10,10') angeordnet sind.

2. Elektrodenanordnung nach Anspruch 1, **dadurch gekennzeichnet, daß** die Schaltmittel (20, 22; 34) ihren Schaltzustand im stromlosen Zustand beibehalten.

3. Elektrodenanordnung nach Anspruch 1 oder 2, **dadurch gekennzeichnet, daß** die Schaltmittel (20, 22; 34) über die Elektrodenleitung (10, 10') ansteuerbar sind.

4. Elektrodenanordnung nach Anspruch 3, **dadurch gekennzeichnet, daß** die Schaltmittel mit mindestens einem Decoder (22) verbunden sind, der seinerseits an mindestens eine elektrische Leitung (18) der Elektrodenleitung (10) angeschlossen ist und über diese Steuersignale empfangen und die Schaltmittel (20) in Abhängigkeit der Steuersignale einzeln ansteuern kann.

5. Elektrodenanordnung nach Anspruch 4, **dadurch gekennzeichnet, daß** die elektrische Leitung (18) eine Zuleitung für elektrische Signale zu und/oder von mindestens einem der elektrisch leitenden Oberflächenbereiche (14, 16) ist.

6. Elektrodenanordnung nach einem der Ansprüche 1 bis 5, **gekennzeichnet durch** einen Energiespeicher (24), der mit den Schaltmitteln (20) und/oder dem Decoder (22) verbunden und mit einer geringeren Leistung über die Elektrodenleitung aufladbar ist, als er an die Schaltmittel (20) und/oder Decoder (22) abgeben kann.

7. Elektrodenanordnung nach Anspruch 6, **dadurch gekennzeichnet, daß** der Energiespeicher ein elektrischer Kondensator (24) ist.

8. Elektrodenanordnung nach einem der Ansprüche 1 bis 7, **dadurch gekennzeichnet, daß** die Schaltelemente derart gestaltet sind, daß zum Ausschalten einer Verbindung zwischen der Elektrodenleitung und einem elektrisch leitenden Oberflächenbereich die Verbindung dauerhaft zerstörbar ist.

9. Elektrodenanordnung nach Anspruch 6 oder 7 und 8, **dadurch gekennzeichnet, daß** der Energiespeicher zur Abgabe der für die Zerstörung der Verbindung benötigten Energie gestaltet ist.

10. Elektrodenanordnung nach einem der Ansprüche 1 bis 9, **dadurch gekennzeichnet, daß** die Elektrodenanordnung Steuermittel (22) enhält, die eingangsseitig über zumindest eine elektrische Leitung (18) für Signale zu und von den elektrisch leitenden Oberflächenbereichen (14, 16) an die kardioelektrische Vorrichtung anschließbar und ausgangsseitig mit den elektrisch leitenden Oberflächenbereichen (14, 16) verbunden sowie derart gestaltet sind, daß sie durch über die elektrische Leitung (18) eingehende Steuersignale derart steuerbar sind, daß einzelne Oberflächenbereiche oder Kombinationen von Oberflächenbereichen mit der kardioelektrischen Vorrichtung zum Abgeben und Aufnehmen von Signalen für das Ermitteln von für die dauerhafte Verbindung mit der kardioelektrischen Vorrichtung geeigneten elektrisch leitenden Oberflächenabschnitten verbindbar sind.

11. Elektrodenanordnung nach Anspruch 1, mit einem relativ zur Elektrodenleitung längs- und/oder drehbeweglich geführten Mandrin (36), **dadurch gekennzeichnet, daß** der Mandrin (36) Betätigungselemente (44) für mechanische Schaltelemente (34) aufweist, mit denen die Verbindung zwischen einzelnen der elektrisch leitenden Oberflächenbereiche und der kardioelektrischen Vorrichtung im Bereich der Elektrodenleitung (10') dauerhaft ein- oder ausschaltbar ist.

12. Elektrodenanordnung nach Anspruch 11, **dadurch gekennzeichnet, daß** die Schaltelemente (34) im Bereich des distalen Endes der Elektrodenleitung (10') längs- oder drehbeweglich angeordnet sind, mit zumindest einer elektrischen Leitung in der Elektrodenleitung in elektrischem Kontakt stehen und Kontaktbereiche, die in einer von zumindest zwei möglichen Stellungen der Schaltelemente einen mit mindestens einem elektrisch leitenden Oberflächenbereich in Verbindung stehenden Gegenkontakt kontaktieren sowie Schaltnocken (44) aufweisen, mit denen ein Schaltelement mittels entsprechender Gegennocken (46) am Mandrin (36) derart mittels des Mandrin (36) bewegbar ist, daß die Schaltmittel (34) mittels des Mandrin (36) jeweils in eine einen Oberflächenbereich mit der kardioelektrischen Vorrichtung verbindende und eine einen Oberflächenbereich von den kardioelektrischen Vorrichtung trennende Stellung bewegbar sind.

## Claims

1. An implantable electrode assembly, comprising an electrode line (10, 10') having an insulating sheath (12) and having several electrically conductive surface areas (14, 16) in the vicinity of the distal end of the electrode line (10, 10') for emitting electrical signals to a heart and/or for receiving signals from a heart, which via the electrode line (10, 10') can be connected electrically with a cardioelectric device receiving electric signals and/or emitting pulses, such as a defibrillator or a cardiac pacemaker, and also having switching means (20, 22; 34), which are configured in such a manner that the connection between individual ones of the electrically conductive surface areas (14, 16) and the cardioelectric device in the vicinity of the electrode line (10, 10') can be permanently connected or disconnected,
**characterised in that** the switching means (20, 22; 34) are disposed inside the electrode line (10, 10').

2. An electrode assembly according to Claim 1,
**characterised in that** the switching means (20, 22; 34) retain their switching state in the current-free condition.

3. An electrode assembly according to Claim 1 or 2,
**characterised in that** the switching means (20, 22; 34) can be controlled via the electrode line (10, 10').

4. An electrode assembly according to Claim 3,
**characterised in that** the switching means are connected to at least one decoder (22), which in turn is connected to at least one electric line (18) of the electrode line (10) and via said line receives control signals and can individually control the switching means (20) in dependence on the control signals.

5. An electrode assembly according to Claim 4,
**characterised in that** the electric line is a lead for electrical signals to and/or from at least one of the electrically conductive surface areas (14, 16).

6. An electrode assembly according to one of Claims 1 to 5,
**characterised by** an energy store (24), which is connected to the switching means (20) and/or the decoder (22) and can be charged with a lower power via the electrode line than it can deliver to the switching means (20) and/or decoder (22).

7. An electrode assembly according to Claim 6,
**characterised in that** the energy store is an electric capacitor.

8. An electrode assembly according to one of Claims 1 to 7,
**characterised in that** the switching elements are configured in such a manner that to disconnect a connection between the electrode line and an electrically conductive surface area, the connection can be permanently destroyed.

9. An electrode assembly according to one of Claims 6 or 7 or 8,
**characterised in that** the energy store is configured to deliver the energy required for destroying the connection.

10. An electrode assembly according to one of Claims 1 to 9,
**characterised in that** the electrode assembly contains control means (22), which at the input side can be connected via at least one electric line (18) for signals to and from the electrically conductive surface areas (14, 16) to the cardioelectric device, and at the output side are connected to the electrically conductive surface areas (14, 16), and are also configured in such a manner that they can be controlled by control signals entering via the electric line (18) in such a manner that individual surface areas or combinations of surface areas can be connected with the cardioelectric device for emitting and receiving signals for determining electrically conductive surface portions suitable for permanent connection to the cardioelectric device.

11. An electrode assembly according to Claim 1, having a stylet (36) guided with longitudinal and/or rotational mobility in relation to the electrode line,
**characterised in that** the stylet (36) comprises operating elements (44) for mechanical switching elements (34), with which the connection between individual ones of the electrically conductive surface areas and the cardioelectric device in the vicinity of the electrode line (10') can be permanently connected or disconnected.

12. An electrode assembly according to Claim 11,
**characterised in that** the switching elements (34) are disposed with longitudinal or rotational mobility in the vicinity of the distal end of the electrode line (10'), are in electrical contact with at least one electric line in the electrode line and contact areas, which in one of at least two possible positions of the switching elements contact a counter-contact communicating with at least one electrically conductive surface area and also comprise control cams (44), with which a switching element can be moved by means of a corresponding counter-cam (46) on the stylet (36) by means of the stylet (36) in such a manner that the switching means (34) can be moved by means of the stylet (36) in each case into a position connecting a surface area with the cardioelectric device and separating a surface area from the cardioelectric device.

## Revendications

1. Dispositif à électrode implantable, comprenant une conduite d'électrode (10, 10') avec une gaine isolante (12) et plusieurs zones de surface conductrices électriques (14, 16) au niveau de l'extrémité distale de la conduite d'électrode (10, 10') pour le débit de signaux électriques à un coeur et/ou pour la réception de signaux émis par un coeur, électriquement connectables au moyen de la conduite d'électrode (10, 10') à un appareil cardio-électrique recevant des signaux électriques et/ou débitant des impulsions tel qu'un défibrillateur ou un pacemaker, et avec des moyens de commutation (20, 22 ; 34) configurés de manière à ce que la connexion entre certaines des zones de surface conductrices électriques (14, 16) et l'appareil cardio-électrique soit continûment activable ou désactivable au niveau de la conduite d'électrode (10, 10'), **caractérisé en ce que** les moyens de commutation (20, 22 ; 34) sont disposés à l'intérieur de la conduite d'électrode (10, 10') .

2. Dispositif à électrode selon la revendication 1, **caractérisé en ce que** les moyens de commutation (20, 22 ; 34) conservent leur état de commutation à l'état sans courant.

3. Dispositif à électrode selon la revendication 1 ou 2, **caractérisé en ce que** les moyens de commutation (20, 22 ; 34) sont commandables par la conduite d'électrode (10, 10').

4. Dispositif à électrode selon la revendication 3, **caractérisé en ce que** les moyens de commutation sont reliés à un décodeur (22) au moins, lequel est à son tour connecté à une ligne électrique (18) au moins de la conduite d'électrode (10) et peut recevoir des signaux de commande au moyen de celle-ci et commander isolément chaque moyen de commutation (20) en fonction des signaux de commande.

5. Dispositif à électrode selon la revendication 4, **caractérisé en ce que** la ligne électrique (18) est un conducteur d'amenée pour des signaux électriques vers et/ou depuis au moins une des zones de surface conductrices électriques (14, 16).

6. Dispositif à électrode selon l'une des revendications 1 à 5, **caractérisé par** un accumulateur (24) connecté aux moyens de commutation (20) et/ou au décodeur (22) et pouvant être chargé à faible puissance au moyen de la conduite d'électrode, de manière à pouvoir débiter du courant aux moyens de commutation (20) et/ou au décodeur (22).

7. Dispositif à électrode selon la revendication 6, **caractérisé en ce que** l'accumulateur est un condensateur électrique (24).

8. Dispositif à électrode selon l'une des revendications 1 à 7, **caractérisé en ce que** les éléments de commutation sont configurés de manière à ce que la connexion soit continûment annulable pour la désactivation d'une connexion entre la conduite d'électrode et une zone de surface conductrice électrique.

9. Dispositif à électrode selon la revendication 6 ou 7 ou 8, **caractérisé en ce que** l'accumulateur est configuré pour le débit du courant nécessité pour l'annulation de la connexion.

10. Dispositif à électrode selon l'une des revendications 1 à 9, **caractérisé en ce que** le dispositif à électrode comprend des moyens de commande (22) connectables à l'appareil cardio-électrique côté entrée au moyen d'au moins une ligne électrique (18) pour des signaux électriques vers et depuis les zones de surface conductrices électriques (14, 16) et connectables côté sortie aux zones de surface conductrices électriques (14, 16), et configurés de manière à être commandables par des signaux de commande conduits par la ligne électrique (18) pour que différentes zones de surface ou combinaisons de zones de surface soient connectables à l'appareil cardio-électrique pour le débit et la réception de signaux afin de déterminer des sections de surface conductrices électriques connectables, adaptées pour la connexion continue à l'appareil cardio-électrique.

11. Dispositif à électrode selon la revendication 1, avec un mandrin (36) guidé de manière à ce que celui-ci soit mobile longitudinalement et/ou en rotation par rapport à la conduite d'électrode, **caractérisé en ce que** le mandrin (36) présente des éléments de manoeuvre (44) pour des- éléments de commutation mécaniques (34) avec lesquels la connexion entre certaines des zones de surface conductrices électriques et l'appareil cardio-électrique est continûment activable ou désactivable au niveau de la conduite d'électrode (10').

12. Dispositif à électrode selon la revendication 11, **caractérisé en ce que** les éléments de commutation (34) au niveau de l'extrémité distale de la conduite d'électrode (10') sont disposés de manière à être mobiles longitudinalement et/ou en rotation, sont en contact électrique avec au moins une ligne électrique dans la conduite d'électrode et présentent des zones de contact, lesquelles, dans une de deux positions possibles au moins des éléments de commutation, contactent un contact opposé connecté à au moins une zone de surface conductrice électrique, ainsi que des ergots de commutation (44) avec lesquels un élément de commutation est mobile au moyen du mandrin (36) grâce à des contre-ergots (46) correspondants sur le mandrin (36) de telle manière que les éléments de commutation (34) soient déplaçables chacun au moyen du mandrin (36) vers une position de connexion d'une zone de surface avec l'appareil cardio-électrique et une position de coupure entre une zone de surface et l'appareil cardio-électrique.
